Europäisches Patentamt

European Patent Office

Office européen des brevets

(19)

(11) Veröffentlichungsnummer: **0 177 910**
**A2**

# (12) EUROPÄISCHE PATENTANMELDUNG

(21) Anmeldenummer: 85112625.0

(22) Anmeldetag: 04.10.85

(51) Int. Cl.⁴: **A 61 K 45/02, A 61 K 45/06**

(30) Priorität: 05.10.84 DE 3436637
05.10.84 DE 3436638
18.06.85 DE 3521733

(43) Veröffentlichungstag der Anmeldung: 16.04.86
Patentblatt 86/16

(84) Benannte Vertragsstaaten: AT BE CH DE FR GB IT LI LU NL SE

(71) Anmelder: BIOFERON Biochemische Substanzen GmbH & Co, Erwin-Rentschler-Strasse 21, D-7958 Laupheim 1 (DE)

(72) Erfinder: Brzoska, Josef, Dr., Hopfenweg 10, D-7958 Laupheim (DE)
Erfinder: v. Eichborn, Johann-Friedrich, Dr., Humlangen 6, D-7901 Hüttisheim (DE)
Erfinder: Obert, Hans-Joachim, Dr., Adolf-Gröber-Strasse 12, D-7958 Laupheim (DE)

(74) Vertreter: Dipl.-Ing. Schwabe, Dr. Dr. Sandmair, Dr. Marx, Stuntzstrasse 16 Postfach 86 02 45, D-8000 München 86 (DE)

(54) Verwendung von Interferon-gamma (IFN-gamma) enthaltenden Präparationen zur systemischen Behandlung von verschiedenen Erkrankungen des Menschen in niedriger Dosierung.

(57) Die Erfindung betrifft die Verwendung von Interferon-gamma enthaltenden Präparationen zur systemischen Behandlung von Erkrankungen des Menschen in der Dosierung von 0,1 bis 2 Millionen Internationale Referenzeinheiten (I.E.) (entspr. ca. 10–200 µg) als Tagesdosis, zu verabreichen in Abständen von täglich bis zu monatlich, bezogen auf einen erwachsenen Patienten von etwa 60 kg Körpergewicht und 1,7 m² Körperoberfläche.

EP 0 177 910 A2

ACTORUM AG

Verwendung von Interferon-gamma (IFN-gamma) enthaltenden
Präparationen zur systemischen Behandlung von verschiedenen
Erkrankungen des Menschen in niedriger Dosierung

Alle bekannten Interferone werden auf Grund von Homologien
sowohl zwischen den Nukleotidsequenzen ihrer Strukturgene als
auch zwischen ihren Aminosäuresequenzen eindeutig in drei Gruppen
eingeteilt, die als IFN-alpha, IFN-beta und IFN-gamma bezeichnet
werden.

Die verwendeten Bezeichnungen folgen der neuesten Empfehlung
des "Interferon Nomenclature Commitee", die von J. Vilcek 1983
in Archives of Virology Vol. 77, S. 283-285, veröffentlicht
wurden. Zusätzlich zu den genannten Hauptkriterien kann zur
Charakterisierung der IFN-gamma-Gruppe herangezogen werden,
daß keine immunologische Kreuzreaktion mit den beiden anderen
Gruppen erfolgt sowie ihre biologische Instabilität bei pH 2
im Gegensatz zu IFN-alpha und IFN-beta.

IFN-gamma wird nach Stimulation der Gesamtpopulation der Leukozyten, nach der Art der Präparation üblicherweise als buffy
coats bezeichnet, durch Mitogene, Antigene oder spezifische
Antikörper in einem komplexen Prozeß zusammen mit einer großen
Anzahl weiterer Faktoren (Mediatoren) ausgeschüttet wie Interleukin 1 (IL 1), Interleukin 2 (IL 2), koloniestimulierender
Faktor (CSF), migrationsinhibierender Faktor (MIF), Makrophagen
aktivierender Faktor (MAF), Tumor nekrotisierender Faktor (TNF)
Lymphotoxin (LT) und Fibroblastenwachstums-Faktor (FGF). Weitere
Faktoren sind beschrieben von J. A. Georgiades et al. in Came,
P. E. und Carter, W.A. (eds.), Interferons and Their Applications,
Springer Verlag, 1984, und von Waksman, B. H. in Cohen, S.
et al. (eds.), Biology of the Lymphokines, Academic Press Inc.,
1979.

Diejenigen Faktoren, die von der Untergruppe der Lymphozyten
gebildet werden, bezeichnet man allgemein als Lymphokine. Zu
dieser Gruppe wird auch IFN-gamma gerechnet, das hauptsächlich
von T-Lymphozyten produziert wird. Die Syntheseleistung der
IFN-gamma produzierenden T-Lymphozyten wird ihrerseits wieder
von dem Vorhandensein der oben erwähnten Faktoren beeinflußt.

Weiterhin existieren etablierte bzw. transformierte Zellinien, die IFN-gamma konstitutiv produzieren, wie von N. Fujii et al. in J. Immunology 130, S. 1683-1686, und A. Zlotnik et al. in J. Immunology 131, S. 794-800, beschrieben.

Ein bevorzugtes Verfahren zur Gewinnung von humanem Roh-IFN-gamma wird nachfolgend beschrieben. Als Ausgangsmaterial dienen lymphozytenreiche Plasmafraktionen von Blutkonserven, sogenannte "buffy coats", die gepoolt und durch schonende Zentrifugation bei 600 - 800 g von Plasmaresten befreit werden. Die pelletierten Gesamtleukozyten werden in vorgewärmtem Medium mit einer Dichte von 5 Millionen Zellen/ml suspendiert. Die Zellsuspension wird in aliquots in geeigneten Kultivierungsgefäßen nach Zugabe eines Mitogens (z.B. Phythämagglutinin) und Phorbolester (z.B. Phorbolmyristylacetat (PMA) bis zu 70 Stunden bei 37 Grad C auf einem Schüttler inkubiert. Die IFN-gamma-haltigen Kulturüberstände werden durch Zentrifugation gewonnen und können bis zur Weiterverwendung bei 4 Grad C gelagert werden. Üblicherweise enthalten die so gewonnenen Präparationen etwa 10.000 Internationale Referenzeinheiten (I.E.) IFN-gamma pro ml.

Der IFN-gamma-Gehalt wird durch einen CPE-Reduktionstest in geeigneten Indikatorzellen (z.B. WISH) mit einem Testvirus (z.B. EMC der Maus) gegen den Referenzstandard Gg 23-901-530 des National Institute of Health (USA) als antivirale Aktivität des Präparates bestimmt.

Weitere Methoden zur Gewinnung von IFN-gamma-Präparationen werden von Y.K. Yip et al. in "Infection and Immunity", Okt. 1981, S. 131- 133, Vol. 34 und in den US-PS 4,376,821 und 4,376,822 sowie 4,460,685 beschrieben.

Ein grundsätzlich anderer Weg zur Gewinnung von humanen IFN-gamma-Präparaten wird mit der Einschleusung des humanen Strukturgens mit Hilfe geeigneter Vektoren in heterologe Wirtszellen beschritten. Diese rekombinanten Gene werden konstitutiv oder nach Zugabe spezifischer Induktoren von der Wirtszelle exprimiert.

Auch wenn es sich bei dem Strukturgen um eine eindeutig definierte Sequenz aus Nukleotiden handelt, die zwingend zur Synthese einer daraus ableitbaren Aminosäuresequenz führt, ist das IFN-gamma-Molekül des mit Hilfe der Wirtszelle hergestellten Präparates nicht notwendigerweise mit einem natürlich vorkommenden IFN-gamma identisch. Diese Möglichkeit der Variation unter Erhaltung der spezifischen Wirkung durch "post processing" wird durch das sogenannte Proteindesign erweitert, durch das das Strukturgen durch chemische bzw. biochemische Vollsynthese oder Modifikation verändert werden kann.

Als Wirtszellen zur Aufnahme des Humangens können außer Bakterien (z.B. E.coli wie von G. Simons et al. in Gene 28, S. 55-64, 1984, beschrieben) auch Hefen (z.B. Saccharomyces cerevisiae, wie von R. Derynck et al. in Nucleic Acids Research 11, S. 1819-1837, 1983, beschrieben) oder eukaryotische Zellen (wie z.B. Chinese Hamster Ovary Cells, wie von S. J. Scahill et al. in Proc. Nat. Acad. Sci. USA 80, S. 4654-4658, 1983, Affenzellen, wie von P. W. Gray et al. in Nature 295, S. 503-508, 1982, beschrieben) dienen.

In einigen dieser Systeme reichert sich entweder IFN-gamma bis zu einer Konzentration von mehr als 100.000 I.E. pro ml in der Kulturflüssigkeit an oder es wird im Wirt selbst angereichert und stellt dort bis zu 25 Prozent des Proteingehalts der Zelle dar.

Die Anreicherung bzw. Reinigung der nach den vorstehend beschriebenen Verfahren erhaltenen IFN-gamma-Präparationen kann nach den folgenden Methoden einzeln oder in Kombination erfolgen:

1. Controlled Pore Glass (CPG) oder Silica-Gel;

2. Gelfiltration (z.B. AcA 54 oder Sephacel S 200);

3. Ionenaustauschchromatographie (CM-Sepharose oder Phosphocellulose oder DEAE-cellulose);

4. Affinitätschromatographie (Con A-Sepharose oder Poly-U-Sepharose oder Cu-Chelat-Sepharose);

5. Immunaffinitätschromatographie (Anti-IFN-gamma-Sepharose);

6. HPLC (z.B. mit Reversed Phase Materialien).

Entsprechende Verfahren wurden von Y.K. Yip et al. "Partial Purification and Characterization of Human Gamma (Immune) Interferon"
in Proc. Nat. Acad. Sci. USA, 78, S. 1601-1605, 1981, oder von
D. Novick et al., EMBO Journal 2, S. 1527-1530, 1983, oder in
der DE-PS 3136166 A 1 beschrieben. .

Durch geeignete Kombinationen ist eine Reinigung bis zur elektrophoretischen Homogenität möglich, z. Zt. liegt die dabei erreichte
maximale spezifische Aktivität bei 100 - 200 Millionen I.E., während
die beschriebenen Durchschnittswerte bei 10 und 50 Millionen I.E.
liegen.

Die Interferone (IFN-alpha, IFN-beta, IFN-gamma) wurden bisher
in sehr unterschiedlichen Dosen und Applikationsweisen bei Patienten
therapeutisch eingesetzt. Ein- oder mehrmalig pro Woche verabreichte
Dosen bis zu ca. 10 Millionen I.E. werden gemeinhin als niedrig,
Dosen ab 20-50 Millionen I.E. als hoch bezeichnet.

In der Anfangszeit der therapeutischen Anwendung der Interferone
standen aufgrund des Fehlens geeigneter biotechnologischer Herstellungsverfahren diese Substanzen lediglich in so geringen Mengen
zur Verfügung, daß nur Dosen von weniger als 3 Millionen I.E.
verabreicht werden konnten. Der Einsatz höherer Dosen ist beim
IFN-alpha und IFN-beta seit Mitte der siebziger Jahre, beim IFN-
gamma seit 1-2 Jahren möglich. Seither werden bei einer systemischen
Behandlung Dosen von weniger als 3 Millionen I.E. in der Regel nur
in Toleranz- und Toxizitätsprüfungen sowie in pharmakokinetischen
Studien eingesetzt.

Für IFN-alpha und IFN-beta gilt, daß bei der systemischen Behandlung
von Tumoren und Viruserkrankungen Dosen bis zu ca. 3 Millionen I.E.
entweder unwirksam oder höheren Dosen unterlegen sind (Tumoren:
Kirkwood, J.M. und Ernstoff, M.S.: J. Clin. Oncol. 2, 336-352, 1984;
Billiau, A.: Contr. Oncol. 20, 251-269, 1984; Bonnem, E.M. und
Spiegel, R.J.: J. Biol. Resp. Modif. 3, 580-598, 1984; Viruserkrankungen: Merigan, T.C. et al.: N. Engl. J. Med. 298, 981-987,
1978; Heidemann, E. et al. Dtsch. Med. Wschr. 107, 695-697, 1982;

Levin, S.: Isr. J. Med. Sci. 19, 955-958, 1983; Billiau, A.: s.o.,
Greenberg, S.B. und Harmon, M.W., Armstrong, J.A. in Came, P.E.
und Carter, W.A.: Interferons and Their Applications. Springer
Verlag, Berlin 1984). Lediglich bei lokaler Behandlung (intratumorale, peritumorale, intraventrikuläre, intrathekale, intraläsionale, periläsionale oder intranasale Applikation) zeigen
Dosen von weniger als 3 Millionen I.E. örtlich eine vergleichbare Wirksamkeit wie eine systemische Applikation höherer Dosen
(Literatur s.o.).

Bei akuten Viruserkrankungen wie Herpes zoster hat sich eine Dosis
von 0,5 Millionen I.E. IFN-alpha oder IFN-beta pro kg Körpergewicht
und Tag bewährt, d.h. bei Erwachsenen täglich ca. 30 Millionen
I.E. (Merigan, T.C.: s.o.; Heidemann, E. et al.: Onkologie 7,
210-212, 1984). Chronische Viruserkrankungen wie die chronisch
aktive Hepatitis B werden in der Regel mit 3-10 Millionen I.E. IFN-
alpha oder IFN-beta täglich oder 3mal wöchentlich behandelt (Müller,
R. et al.: Z. Gastroenterologie 20, 105-109, 1982; Billiau, A.: s.o.;
Levin, S.: s.o.). Bei der Multiplen Sklerose und Amyotrophen Lateralsklerose, bei denen eine Virusätiologie diskutiert wird, wurden
täglich 6 Millionen bzw. 100-200 Millionen I.E. IFN-alpha systemisch
verabreicht (Knobler, R.L. et al.: Neurology 34, 1273-1279, 1984;
Färkkilä, M.A. et al.: Act. Neur. Sci. 69, 184-185, 1984).

Aufgrund von In-vitro-Untersuchungen gilt IFN-gamma als weniger
antiviral wirksam als IFN-alpha oder IFN-beta (z.B. Munoz, A.,
Carrasco, L.: FEMS Microbiol. Letters 21, 105-111, 1984). Aus
diesem Grunde wurde IFN-gamma für die systemische Behandlung von
Viruserkrankungen bislang nicht verwendet.

Patienten mit Tumoren wurden bisher lediglich im Rahmen von Phase-
I-Studien behandelt, so daß eine Aussage über eine therapeutische
Wirksamkeit von IFN-gamma bei diesen Erkrankungen nicht möglich
ist (Yamazaki, S.: Jpn. J. Med. Sci. Biol. 37, 209-223, 1984;
Sherwin, S.A. et al.: J. Biol. Resp. Modif. 3, 599-607, 1984;
Guttermann, J.U. et al.: Cancer Res. 44, 4164-4171, 1984; Niederle
et al. in Kirchner, H. und Schellekens H.: The Biology of the
Interferon System 1984, Elsevier, Amsterdam 1985).

Bei Psoriasis, allergischen Erkrankungen, M. Crohn, Multipler
Sklerose, Amyotropher Lateralsklerose und Schmerzen wurde IFN-gamma
bisher nicht eingesetzt.

In eigenen klinischen Studien zur therapeutischen Wirksamkeit
von IFN-gamma wurden Patienten mit unterschiedlichen Erkrankungen
behandelt. Um die optimal wirksame Dosis von IFN-gamma zu ermitteln,
wurden Dosen von 50.000 I.E. bis zu 100 Millionen I.E. ein- oder
mehrmalig pro Woche systemisch verabreicht. Bei diesen Studien
zeigte sich überraschend, daß IFN-gamma im Gegensatz zu IFN-alpha
oder IFN-beta bei sehr verschiedenen Erkrankungen therapeutisch
wirksam war und die effektivste Dosis von IFN-gamma bei allen
Erkrankungen in dem sehr niedrigen Bereich von 0,1 bis 2 Millionen
I.E. bzw. ca. 10 - 200 µg lag. Bei den Patienten, die mit diesen
niedrigen Dosen behandelt wurden, war nämlich häufiger eine Wirkung
des IFN-gamma festzustellen als bei denen, die höhere Dosen erhielten. Die Überlegenheit der sehr niedrigen über die höheren
Dosen zeigte sich bei 2 Patienten besonders eindrucksvoll: Der
therapeutische Effekt des IFN-gamma verschwand, als bei ihnen
die Dosis erhöht wurde (Beispiel 1,2).

Die Wirksamkeit von IFN-gamma in der niedrigen Dosierung zeigte
sich bei unterschiedlichen Tumoren wie Hypernephrom (Beispiel
Nr. 1-3), Pseudomyxoma (Nr. 4), Mastozytose (Nr. 5), Immunocytom
(Nr. 6), Melanom (Nr. 7), M. Hodgkin (Nr. 8), Schmincke-Tumor
(Nr. 9), Synovial-Sarkom (Nr. 10) und Pankreas-Karzinom (Nr. 11).

Insbesondere überraschte die Wirksamkeit bei verschiedenen Viruserkrankungen wie chronisch aktive Hepatitis B (Beispiel Nr. 12),
Herpes zoster (Nr. 13, 14) und Kondylomen (Nr. 15). IFN-gamma
zeigte bei diesen Erkrankungen nämlich trotz der geringen In-vitro-
Aktivität nicht nur in gleicher Dosierung wie IFN-alpha oder IFN-
beta, sondern sogar in wesentlich niedrigerer Dosierung als die
beiden anderen Interferontypen einen therapeutischen Effekt.

Ebenfalls unerwartet war IFN-gamma wirksam bei folgenden Erkrankungen: Psoriasis (Beispiel Nr. 3), Allergien (Nr. 16, 20), M.
Crohn (Nr. 17), Amyotrophe Lateralsklerose (Nr. 18), Multiple
Sklerose (Nr. 19) und Schmerzen (Nr. 1, 2, 3, 11).

Für die systemische Behandlung können natürliches IFN-gamma und rekombinantes IFN-gamma wie folgt dosiert werden: intravenös als Bolus oder über mehrere Minuten, Stunden, Tage oder Wochen, intramuskulär und/oder subkutan. Auch bei intratumoraler, peritumoraler, intraläsionaler bzw. periläsionaler Applikation wird neben der lokalen auch eine systemische Wirkung erzielt. Bei allen Applikationsformen können auch die dem Fachmann bekannten Depotformen verwendet werden, z.B. Mikro- und Nanokapseln, Liposomen und Pflaster. Eine Dosis von 0,1 Millionen I.E. bis zu 2 Millionen I.E. bzw. 10 µg bis 200 µg kann pro Tag einmalig gegeben werden oder portioniert mehrmalig. Bei mehrtägiger Gabe kann die Dosis von $0,1-2,0 \times 10^6$ I.E. bzw. ca. 10-200 µg wie folgt verabreicht werden:

a) an aufeinanderfolgenden Tagen
b) alle 2 bis 6 Tage
c) 1mal pro Woche
d) alle 2 bis 4 Wochen
e) 1mal pro Monat
f) jedesmal bei Verschlechterung des Krankheitsbildes.

Die Dosis von $0,1-2,0 \times 10^6$ I.E. bzw. ca. 10 µg bis 200 µg bezieht sich auf einen Patienten von 60 kg Körpergewicht und 170 cm Körpergröße, entsprechend 1,74 qm Körperoberfläche (Dubois u. Dubois, Arch. intern. Med. 17, 863, 1916). Entsprechend wird für den einzelnen Patienten die individuelle Dosis ermittelt, d. h. $0,06-1,2 \times 10^6$ I.E. bzw. 6-120 µg pro $m^2$ Körperoberfläche.

Zur Steigerung der Effektivität der Interferon-gamma-Präparationen können folgende Substanzen zusätzlich gegeben werden:

a) Verabreichung von anderen Interferonen und/oder anderen von Leukozyten gebildeten bzw. durch gentechnologische Verfahren hergestellten Zell-Mediatoren wie IL 1, IL 2, CSF, MIF, MAF, TNF, LT und FGF;

b) Verabreichung von in der Therapie von Tumoren, Viruserkrankungen, Psoriasis, allergischen Erkrankungen, Morbus Crohn, Amyotropher Lateralsklerose, Multipler Sklerose und Schmerzen bisher verwendete Substanzen wie Alkylanzien, Folsäureantagonisten, Antimetabolite des Nukleinsäurestoffwechsels, Spindelgifte, Antibiotika, Immuntherapeutika, Pyrimidinanaloga,

Purinnukleoside, Amine, Triazolnukleoside, Kortikoide, Kalzium, Antihistaminika, Retinoide, lichtsensibilisierende Substanzen, Inhibitoren der Lipoxygenase bzw. Cyclooxygenase, Fumarsäure und deren Salze, Analgetika, Psychopharmaka, Lokalanästhetika, Spasmolytika, Antirheumatika, Kalzium-Antagonisten und Betablocker.

Detaillierte Angaben hierzu werden gemacht und weitere Substanzen sind beschrieben von UICC (Hrsg.) in Klinische Onkologie, Springer Verlag, 1982, von J. Fischer (Hrsg.) in Taschenbuch der Onkologie, Urban und Schwarzenberg, 1983, von E. De Clercq in Biochem. J. 205, S. 1-13, 1982, von K.G. Nicholson in Lancet ii, S. 503-506, S. 562-564, S. 617-621, S. 677-682, S. 736-739, 1984, von Braun-Falco, O. et al.: Dermatologie und Venerologie. 3. Aufl. Springer Verlag, Berlin 1984, von Nietsch, P.: Schmerztherapie aktuell, Folge 1-10, medwelt 35, 1984, von Senn, H.J. und Glaus, A.: medwelt 35, 1235-1240, 1984 und von Foley, K.M.: N. Engl. J. Med. 313, 84-95, 1985.

c) Radiologische Maßnahmen wie Bestrahlung oder Einführen radioaktiver Substanzen wie beschrieben von UICC (Hrsg.) in Klinische Onkologie, Springer Verlag, 1982.

Zur Herstellung der jeweiligen Darreichungsform werden die dem Fachmann bekannten Hilfsstoffe verwendet, z.B. Serumeiweißstoffe wie humanes Serumalbumin, Puffersubstanzen wie Phosphate, Glycin, Sorbinsäure, Kaliumsorbat, Partialglyceridgemische gesättigter Pflanzenfettsäuren, Wasser sowie Salze bzw. Elektrolyte wie Protamin-sulfat, Dinatriumhydrogenphosphat, Kaliumhydrogenphosphat, Natrium-chlorid und Zn-Salze.

## BEISPIEL 1

Patient:              G. S., männlich, 57 J., 91 kg, 182 cm

Diagnose:             Hypernephrom mit Knochenmetastase im
                      rechten Oberarmschaft
                      starke Schmerzen und geringe Beweglichkeit
                      des rechten Armes

Substanz:             humane IFN-gamma-Präparation aus E.coli

Applikationsweise:    intramuskulär

Therapieschema:       100 µg 4mal pro Woche über 2 Wochen, danach
                      Erhöhung der Dosis auf 200, 400, 800 und
                      1 600 µg
                      anschließend 2 100 µg 1mal pro Woche über
                      4 Wochen

Ergebnis:             Nach der 1. Injektion von 100 µg waren
                      die Schmerzen deutlich verringert, und
                      der Patient konnte den Arm wesentlich
                      besser bewegen, nach der 8. Injektion
                      von 100 µg hatte er keine Schmerzen mehr.
                      Vier Tage nach der 1. Injektion von 2 100 µg
                      traten wieder Schmerzen im Oberarm auf.

## BEISPIEL 2

Patient:              P. H., männlich, 51 J., 84 kg, 182 cm

Diagnose:             Hypernephrom mit pulmonalen und ossären
                      Metastasen
                      Schmerzen in der Beckenregion und dem
                      Lendenwirbelsäulen-Kreuzbein-Bereich

Substanz:             humane IFN-gamma-Präparation aus E.coli

Applikationsweise: intramuskulär

Therapieschema: 100 µg 4mal pro Woche über 2 Wochen, danach Erhöhung der Dosis auf 200 µg

Ergebnis: Nach der 2. Injektion von 100 µg waren die Schmerzen deutlich verringert und nahmen nach der 3. Injektion von 100 µg weiter ab. Nach der 4. Injektion von 200 µg traten die Schmerzen wieder zunehmend auf.

## BEISPIEL 3

Patient: W. R., männlich, 74 J., 64 kg, 170 cm

Diagnose: Hypernephrom mit cutanen, pulmonalen und ossären Metastasen
Psoriasis vulgaris

Substanz: humane IFN-gamma-Präparation aus E.coli und Difluoromethylornithin (DFMO)

Applikationsweise: IFN-gamma intravenös, DFMO oral

Therapieschema: IFN-gamma zunächst täglich 50 µg über 10 Tage, dann täglich 100 µg über 7 Tage, DFMO täglich 3 x 4 g

Ergebnis: Am 7. Tag gingen die Schmerzen zurück, am 14. Tag war der Patient vollkommen schmerzfrei. Ab dem 12. Tag bildete sich die Psoriasis vulgaris zurück und war bei Therapieende annähernd komplett verschwunden.
Bei der Entlassung des Patienten nach 17 Therapietagen war eine partielle Rückbildung der cutanen und der Lebermetastasen nachweisbar. Der Allgemeinzustand des Patienten hatte sich deutlich gebessert.

0177910

## BEISPIEL 4

Patient:          F. D., männlich, 46 J., 64 kg, 183 cm

Diagnose:        Pseudomyxoma peritonei
mit 4 Fisteln in der Bauchwand

Substanz:        humane IFN-gamma-Präparation aus E.coli

Applikationsweise: intramuskulär

Therapieschema:  100 µg 4mal pro Woche über 2 Wochen

Ergebnis:        Nach der 3. Injektion von 100 µg hatten
sich 2 von 4 Fisteln geschlossen.

## BEISPIEL 5

Patient:          K. W., männlich, 59 J., 73 kg, 178 cm

Diagnose:        maligne Mastozytose mit diffuser Knochenmarks- und Leberinfiltration und extramedullärer Blutbildung

Substanz:        humane IFN-gamma-Präparation aus E.coli

Applikationsweise: intravenös

Therapieschema:  50 µg 5mal pro Woche über 5 Wochen

Ergebnis:        Nach 5 Wochen Therapie zeigte sich eine
Volumenabnahme der vergrößerten Leber
und ein Absinken der Alkalischen Phosphatase von 1 700 E/1 auf 1 000 E/1 (Normbereich bis ca. 190 E/1).

## BEISPIEL 6

Patient:                U. F., 54 Jahre

Diagnose:               progredientes polymorphzelliges Immunocytom

Substanz:               humane IFN-gamma-Präparation aus E.coli

Applikationsweise: intravenös

Therapieschema:         50 µg 5mal pro Woche über 4 Wochen

Ergebnis:               Nach 2 Wochen Therapie war die Erkrankung
                        nicht mehr progredient, nach weiteren
                        2 Wochen gingen die Lymphknotenschwellungen
                        zurück.

## BEISPIEL 7

Patient:                H. E., männlich
Diagnose:               faustgroße Haut-Metastase eines malignen
                        Melanoms, Primärtumor chirurgisch entfernt

Substanz:               humane IFN-gamma-Präparation aus E.coli

Applikationsweise: intravenös

Therapieschema:         100 µg 3-5x/Woche über 5 Wochen

Ergebnis:               Nach 3 Wochen Therapie hatte sich die
                        Metastase um 90 % verkleinert und blieb
                        dann stabil.

0177910

## BEISPIEL 8

Patient:                G. K., männlich, 50 J., 85 kg, 172 cm

Diagnose:               M. Hodgkin Stadium IVb, Metastasen in
                        Leber, Lunge, Knochenmark und abdominalen
                        Lymphknoten

Substanz:               humane IFN-gamma-Präparation aus menschlichen Leukozyten

Applikationsweise:      zunächst subkutan, dann intravenös

Therapieschema:         täglich über 3 Wochen, Dosis von $0,01x10^6$
                        auf $1,0x10^6$ I.E. steigernd

Ergebnis:               Bei Therapieende war eine deutliche Ver-
                        kleinerung der Lebermetastasen festzu-
                        stellen. Außerdem hatte sich die B-Symptomatik verbessert.

## BEISPIEL 9

Patient:                G. P., männlich, 61 J.

Diagnose:               Schmincke-Tumor mit Rezidiv der Lungen-
                        Metastasen

Substanz:               humane IFN-gamma-Präparation aus menschlichen Leukozyten

Applikationsweise:      intravenös

Therapieschema:         $0,5x10^6$ I.E. täglich außer samstags über
                        12 Wochen

Ergebnis:               Nach der 12wöchigen Therapie war eine
                        partielle Remission des ausgedehnten Rezidivs der Lungenmetastasen festzustellen.
                        Der Patient konnte aus der Klinik entlassen
                        werden.

0177910

## BEISPIEL 10

Patient:             B.C., weiblich, 36 J., 52 kg

Diagnose:            Synovial-Sarkom mit pulmonalen und pleuralen
                     Metastasen sowie Pleuraerguß
                     Primärtumor durch Amputation des Beins
                     entfernt

Substanz:            humane IFN-gamma-Präparation aus E.coli

Applikationsweise: intravenös

Therapieschema:      täglich 50 µg

Ergebnis:            Nach 14 Therapietagen war der Pleuraerguß
                     verschwunden.

## BEISPIEL 11

Patient:             H.K., männlich, 86 kg, 181 cm

Diagnose:            Pankreas-Karzinom mit Lebermetastasen

Substanz:            humane IFN-gamma-Präparation aus E.coli

Applikationsweise: intravenös

Therapieschema:      täglich 100 µg

Ergebnis:            Ab dem 2. Tag trat eine deutliche Linderung
                     der Tumorschmerzen ein. Der Patient benötigte
                     deutlich geringere Mengen an starken Anal-
                     getika.

-15-

## BEISPIEL 12

Patient:             M. K., weiblich, 39 J.

Diagnose:            chronisch aktive Hepatitis B

Substanz:            humane IFN-gamma-Präparation aus menschlichen Leukozyten

Applikationsweise:   intravenös

Therapieschema:      5mal pro Woche über 10 Wochen, Dosis von
                     $0,05 \times 10^6$ auf $2,0 \times 10^6$ I.E. steigernd

Ergebnis:            Unter der Therapie sank die DNAP von 17 422
                     auf 5 546 ab.

## BEISPIEL 13

Patient:             E. S., weiblich

Diagnose:            Zoster oticus mit Hörstörung und Facialisparese

Substanz:            humane IFN-gamma-Präparation aus Leukozyten

Applikationsweise:   intramuskulär

Therapieschema:      $0,5 \times 10^6$ I.E. 2mal pro Tag über 5 Tage

Ergebnis:            Nach 5 Tagen Therapie war der Zoster nahezu
                     abgeheilt, und die Funktion des Nervs
                     hatte sich normalisiert.

## BEISPIEL 14

Patient:               T. S., männlich, 51 J.

Diagnose:              Zoster oticus mit Facialisparese

Substanz:              humane IFN-gamma-Präparation aus E.coli

Applikationsweise:     intramuskulär

Therapieschema:        100 µg 2mal pro Tag über 5 Tage

Ergebnis:              Nach 5 Tagen Therapie war der Zoster nahezu abgeheilt, und die Funktion des Nervs hatte sich normalisiert.


## BEISPIEL 15

Patient:               N. N., weiblich, 20 J.

Diagnose:              Condylomata acuminata

Substanz:              humane IFN-gamma-Präparation aus E.coli

Applikationsweise:     subcutan

Therapieschema:        täglich 200 µg über 7 Tage

Ergebnis:              Innerhalb von 3 Wochen nach Ende der Behandlung waren die Kondylome nahezu vollständig verschwunden.

0177910

## BEISPIEL 16

| | |
|---|---|
| Patient: | I. K., weiblich, 56 J. |
| Diagnose: | Asthma bronchiale |
| Substanz: | humane IFN-gamma-Präparation |
| Applikationsweise: | subcutan |
| Therapieschema: | 3 Injektionen im Abstand von 3 Tagen mit jeweils 100 µg |
| Ergebnis: | Nach der Therapie war die Atemnot der Patientin deutlich verringert. Die Medikation mit Kortikosteroiden war nicht mehr notwendig. Die Gabe von Theophyllin konnte in der Dosis auf die Hälfte reduziert werden. |

## BEISPIEL 17

| | |
|---|---|
| Patient: | I. B., männlich, 44 J., 57 kg |
| Diagnose: | M. Crohn |
| Substanz: | humane IFN-gamma-Präparation aus menschlichen Leukozyten |
| Applikationsweise: | intramuskulär oder intravenös |
| Therapieschema: | 3-4mal pro Woche über 8 Wochen, Dosis von 0,02 auf $0,5 \times 10^6$ I.E. steigernd |
| Ergebnis: | Der Crohnindex war durch die Therapie von 290 auf 59 gefallen. (In den Crohnindex gehen mehrere Faktoren ein u.a. Anzahl der Stühle, Bauchschmerzen, Allgemeinbefinden, Gewicht. Diese werden mit Punkten bewertet: Je höher die Punktzahl ist, desto ausgeprägter ist die Erkrankung.) |

## BEISPIEL 18

Patient:               W. W., männlich, 61 J., 65 kg, 175 cm

Diagnose:            Amyotrophe Lateralsklerose

Substanz:            humane IFN-gamma-Präparation aus menschlichen Leukozyten Applikationsweise: intramuskulär

Therapieschema:    3mal pro Woche über 4 Wochen, Dosis von $0,1 \times 10^6$ auf $1 \times 10^6$ I.E. steigernd. Danach 1mal pro Woche $0,5 \times 10^6$ I.E. über 4 Wochen.

Ergebnis:           Unter Therapie besserten sich die Funktionen der oberen Extremität. Die Hände konnten wieder gebeugt und gestreckt werden, auch waren mäßige Streckungen und Beugungen sowie geringfügige Rotationen im Ellenbogengelenk möglich. Beim Schultergelenk war wieder eine geringe Elevation möglich.

## BEISPIEL 19

Patient:               N. F., männlich, 38 J., 53 kg, 169 cm

Diagnose:            Multiple Sklerose

Substanz:            humane IFN-gamma-Präparation aus E.coli

Applikationsweise: subcutan

Therapieschema:    3mal pro Woche 10 µg

Ergebnis:           Nach 6 Wochen waren die Funktionen der oberen und unteren Extremitäten gebessert. Die Hyperreflexie der Arme und der Beine war verschwunden. Die Areflexie des Oberbauches besserte sich, die Schwindelanfälle verschwanden, Morgensteife war nicht mehr zu beobachten.

## BEISPIEL 20

Patient:                C. L., weiblich, 43 J.

Diagnose:               Allergie gegen Apfelsinen seit mehreren
                        Jahren

Substanz:               humane IFN-gamma-Präparation aus menschlichen
                        Leukozyten

Applikationsweise:      subkutan

Therapieschema:         $0,1 \times 10^6$ I.E. einmal pro Woche über 4
                        Wochen

Ergebnis:               Bereits nach der ersten Injektion war
                        die Allergie verschwunden. Die Besserung
                        hielt 6 Monate nach Therapieende an.

**P a t e n t a n s p r ü c h e**

1. Verwendung von Interferon-gamma enthaltenden Präparationen zur systemischen Behandlung von Erkrankungen des Menschen in der Dosierung von ca. 0,1 bis 2 Millionen Internationale Referenzeinheiten (I.E.) bzw. ca. 10 - 200 µg) als Tagesdosis, zu verabreichen in Abständen von täglich bis zu monatlich, bezogen auf einen erwachsenen Patienten von etwa 60 kg Körpergewicht und 1,7 m$^2$ Körperoberfläche.

2. Verwendung nach Anspruch 1 zur Behandlung von Tumoren (solide Tumoren und maligne hämatologische Systemerkrankungen).

3. Verwendung nach Anspruch 1 zur Rezidivprophylaxe von Tumoren.

4. Verwendung nach Anspruch 1 zur Behandlung akuter Viruserkrankungen.

5. Verwendung nach Anspruch 1 zur Behandlung chronischer Viruserkrankungen.

6. Verwendung nach Anspruch 1 zur Prophylaxe von akuten Viruserkrankungen.

7. Verwendung nach Anspruch 1 zur Prophylaxe von chronischen Viruserkrankungen.

8. Verwendung nach Anspruch 1 zur Behandlung von durch humane Papillomviren (HPV) bedingte Erkrankungen.

9. Verwendung nach Anspruch 8 zur Behandlung von Condylomata acuminata.

10. Verwendung nach Anspruch 1 zur Behandlung psoriatischer Erkrankungen.

11. Verwendung nach Anspruch 10 zur Behandlung von Psoriasis vulgaris.

12. Verwendung nach Anspruch 1 zur Behandlung von allergischen Erkrankungen.

13. Verwendung nach Anspruch 12 zur Behandlung von Asthma bronchiale.

14. Verwendung nach Anspruch 1 zur Behandlung von Morbus Crohn.

15. Verwendung nach Anspruch 1 zur Behandlung der Amyotrophen Lateralsklerose.

16. Verwendung nach Anspruch 1 zur Behandlung von Multipler Sklerose.

17. Verwendung nach Anspruch 1 zur Behandlung von Schmerzen.

18. Verwendung nach Anspruch 17 zur Behandlung von tumorbedingten Schmerzen.

19. Verwendung nach Anspruch 1, dadurch gekennzeichnet, daß Interferon-gamma von menschlichen Zellen oder durch gentechnologische Verfahren produziert wird.

20. Verwendung nach Anspruch 1, dadurch gekennzeichnet, daß die Interferon-gamma enthaltende Präparation zusätzlich andere Interferone und/oder andere durch Leukozyten gebildete bzw. durch gentechnologische Verfahren hergestellte Zell-Mediatoren enthält.

21. Verwendung nach Anspruch 1, dadurch gekennzeichnet, daß zusätzlich andere bisher in der Therapie von Tumoren, Viruserkrankungen, Psoriasis, Allergien, Morbus Crohn, Amyotropher Lateralsklerose, Multipler Sklerose und Schmerzen verwendete Substanzen verabreicht werden.

22. Verwendung von Interferon-gamma enthaltenden Präparationen nach einem der Ansprüche 1 bis 18, dadurch gekennzeichnet, daß die Präparation in einer intravenös, intramuskulär oder subkutan zu verabreichenden Applikationsform vorliegt.

23. Verwendung nach Anspruch 22, dadurch gekennzeichnet, daß die dem Fachmann für Polypeptidtherapeutika bekannten Depotformen verabreicht werden.